# EUROPEAN PATENT APPLICATION

(11) **EP 2 684 872 A1**
(43) Date of publication of application: **15.01.2014**
(21) Application number: 12755296.6
(22) Date of filing: 08.02.2012
(51) Int. Cl.: C07D 209/14, C09B 23/00, H01L 31/04, H01M 14/00

(54) **COMPOUND HAVING HEPTAMETHINE STRUCTURE, SENSITIZING DYE AND PHOTOELECTRIC CONVERSION ELEMENT**

(30) Priority: 10.03.2011 JP 2011052873; 10.03.2011 JP 2011052874; 06.07.2011 JP 2011150158
(71) Applicant: Gifu University, Gifu-shi Gifu 501-1193 (JP); Dai-Ichi Kogyo Seiyaku Co., Ltd., Kyoto-shi, Kyoto 600-8873 (JP)
(72) Inventor: SAITO, Yasuteru, Kyoto-shi Kyoto 600-8873 (JP); FUNABIKI, Kazumasa, Gufu-shi Gifu 501-1193 (JP); HIBINO, Atsuhiko, Gufu-shi Gifu 501-1193 (JP); TANAKA, Nagisa, Gufu-shi Gifu 501-1193 (JP)
(74) Representative: Enomoto, Kéi
(86) International application number: PCT/JP2012/000841
(87) International publication number: WO 2012/120784

(57) **Abstract**

Provided is a compound having a heptamethine structure represented by the following general formula (1) used as a sensitizing dye for a photoelectric conversion device, and a photoelectric conversion device using the compound as a sensitizing dye, which may solve the problems of the related art. In the general formula (1), R¹ and R² each represent a hydrogen atom, an alkyl group or an aryl group; R³ to R⁶ each represent an alkyl group having from 1 to 18 carbon atoms; X represents a halogen atom; Y represents a monovalent anion; Z represents an alkyl group, an alkoxy group or an aryl group when R³ to R⁶ each have from 1 to 5 carbon atoms, or a hydrogen atom, an alkyl group, an alkoxy group or an aryl group when R³ to R⁶ each have from 6 to 18 carbon atoms; and m and n each represent an integer of from 1 to 12.

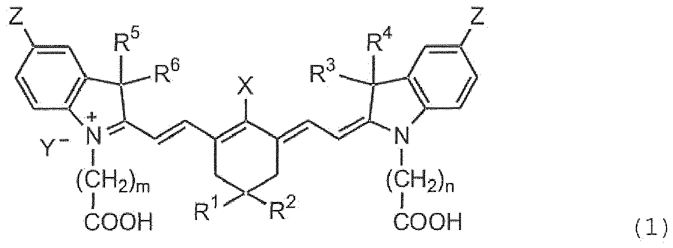

## Description

### BACKGROUND OF THE INVENTION

### Field of the Invention

The present invention relates to a near infrared ray absorbing dye for a dye sensitized solar cell using a semiconductor, and a photoelectric conversion device.

### Background Art

In recent years, various solar cells have been proposed as a photoelectric conversion device to convert light energy to electric energy. Among these, a dye sensitized solar cell, which was announced by M. Grätzel, et al. at Swiss Federal Institute of Technology in Lausanne in 1991, is expected to be a practical low-cost solar cell due to the low-cost materials and process thereof (see Nature, vol. 353, pp. 737-740 (1991)).

The dye sensitized solar cell is generally constituted by a semiconductor electrode, which has a photoelectric conversion layer containing a semiconductor which has absorbed a sensitizing dye on a conductive substrate, a counter electrode, which is provided to face the semiconductor electrode and has a catalyst layer formed on a conductive substrate, and an electrolyte layer, which is held between the semiconductor electrode and the counter electrode.

As a known sensitizing dye for the dye sensitized solar cell, a ruthenium complex dye shown in Nature, vol. 353, pp. 737-740 (1991) and the like have been known as a sensitizing dye that absorbs visible light over a wide wavelength range and has excellent photoelectric conversion capability, i.e., electric power generation capability. However, the ruthenium complex dye is expensive, and inexpensive organic dyes are being developed. In particular, an organic dye that absorbs a near infrared ray is receiving attention since the combination use thereof with an organic dye that absorbs visible light may enhance the capability of the photoelectric conversion device.

For example, JP-A-2000-195570 discloses the use of an organic dye having a polymethine structure as a sensitizing dye, but the photoelectric conversion capability thereof is still insufficient.

JP-A-2007-220412 discloses an organic dye having a heptamethine structure as a sensitizing dye, and also discloses that the use of the organic dye with an iodine redox pair as an electrolyte provides a photoelectric conversion device having excellent capability. However, the organic dye has problems: for example, the organic dye fails to provide sufficient photoelectric conversion capability in a photoelectric conversion device that uses a redox pair other than an iodine redox pair: and even in a photoelectric conversion device using an iodine redox pair, the capability of the device starts deteriorating in a relatively early stage after the production thereof. Accordingly, there is a demand for a near infrared ray absorbing dye that is more excellent in electric power generation capability.

### SUMMARY OF THE INVENTION

The invention has been made under the circumstances as described above, and an object of the invention is to provide a novel compound having a heptamethine structure that absorbs light in the near infrared range and is capable of being used as a sensitizing dye of a photoelectric conversion device, and to provide a photoelectric conversion device that uses the compound as a sensitizing dye and is excellent in photoelectric conversion capability and stability thereof as compared to ordinary photoelectric conversion devices.

The invention relates in one embodiment to a compound having a heptamethine structure represented by the following general formula (1):

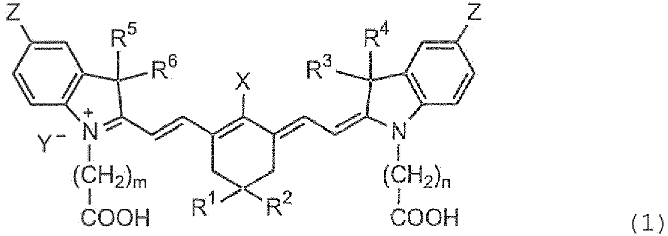

wherein R¹ and R², which may be the same as or different from each other, each represent a hydrogen atom, an alkyl group or an aryl group; R³ to R⁶, which may be the same as or different from each other, each represent an alkyl group having from 1 to 18 carbon atoms; X represents a halogen atom; Y represents a monovalent anion; Z represents an alkyl group, an alkoxy group or an aryl group when R³ to R⁶ each have from 1 to 5 carbon atoms, or a hydrogen atom, an alkyl group, an alkoxy group or an aryl group when R³ to R⁶ each have from 6 to 18 carbon atoms; and m and n, which may be the same as or different from each other, each represent an integer of from 1 to 12.

The invention also relates in another embodiment to a sensitizing dye containing a compound (A) represented by the general formula (1) wherein R³ to R⁶ each represent an alkyl group having from 1 to 12 carbon atoms; and Z represents an alkyl group, an alkoxy group or an aryl group, or a compound (B) represented by the general formula (1) wherein R³ to R⁶ each represent an alkyl group having from 6 to 18 carbon atoms; and Z represents a hydrogen atom, an alkyl group, an alkoxy group or an aryl group.

The invention also relates in still another embodiment to a photoelectric conversion device containing a semiconductor electrode, a counter electrode, and an electrolyte layer that is held between the electrodes, the semiconductor electrode containing the sensitizing dye of the invention adsorbed thereon.

The invention also relates in a further embodiment to a dye sensitized solar cell containing the photoelectric conversion device of the invention.

The compound having a heptamethine structure of the invention may be favorably used as a sensitizing dye of a photoelectric conversion device, such as a dye sensitized solar cell, and may provide such advantageous effects that the conversion efficiency is enhanced as compared to ordinary compounds in a system using a non-iodine redox pair as an electrolyte, and the initial device capability is maintained long after the production of the device in a system using an iodine redox pair as an electrolyte.

### BRIEF DESCRIPTION OF THE DRAWING

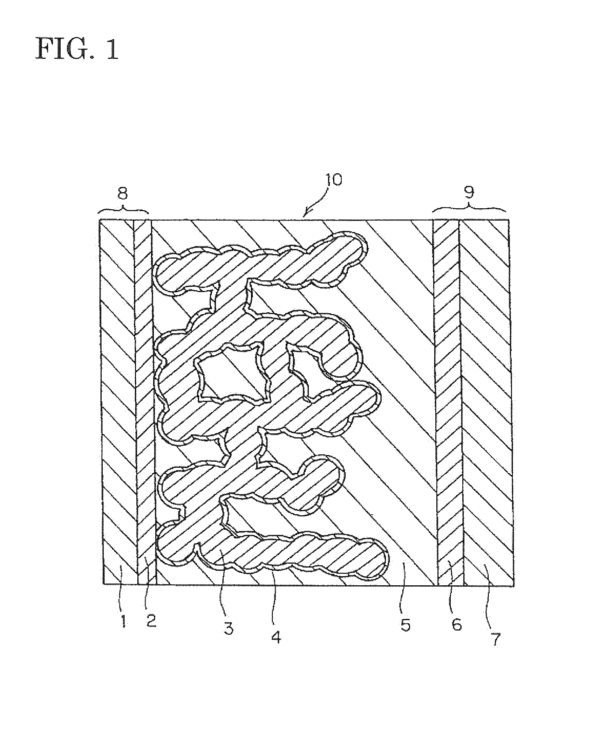

Fig. 1 is a schematic cross sectional view showing an example of a basic structure of a photoelectric conversion device according to one embodiment of the invention.

### DETAILED DESCRIPTION OF THE INVENTION

### 1. Compound represented by General Formula (1)

The compound having a heptamethine structure of the invention has a structure represented by the general formula (1), and in the case where the compound is used as a sensitizing dye of a photoelectric conversion device, such as a solar cell, R¹ to R⁶, X, Y, Z, m and n in the general formula (1) are preferably as follows.

R¹ and R² each represent a hydrogen atom, an alkyl group or an aryl group, in which the alkyl group preferably has from 1 to 12 carbon atoms, and more preferably from 1 to 4 carbon atoms, and the aryl group preferably has from 6 to 12 carbon atoms.

R³ to R⁶ in the compound (A) each preferably represent an alkyl group having from 1 to 12 carbon atoms, and more preferably from 4 to 8 carbon atoms.

In the compound (B), R¹ and R² may be the same as in the compound (A), and R³ to R⁶ each preferably represent an alkyl group having from 6 to 18 carbon atoms, and more preferably from 8 to 12 carbon atoms.

X represents a halogen atom, and preferably a chlorine atom or a bromine atom.

Y represents a monovalent anion, preferably a halogen ion, and more preferably an iodide ion. It is considered that this is because a larger anion may suppress association of molecules.

The alkyl group represented by Z preferably has from 1 to 20 carbon atoms, and more preferably from 3 to 12 carbon atoms, the alkoxy group represented by Z preferably has from 1 to 4 carbon atoms, and the aryl group represented by Z preferably has from 6 to 12 carbon atoms. In the compound (B), Z also preferably represents a hydrogen atom.

m and n each represent an integer of preferably from 1 to 12, more preferably from 1 to 4, and further more preferably 2.

A photoelectric conversion device using the compound represented by the general formula (1) as a sensitizing dye has a photoelectric conversion capability that is enhanced as compared to the photoelectric conversion device which is specifically disclosed in JP-A-2007-220412, and is largely suppressed in time-lapse deterioration of the device capability. It is considered that this is because the compound represented by the general formula (1) has a heptamethine structure close to a planar structure, which facilitates association of molecules due to π-π stacking caused by π electrons, but the compound (A) of the invention contains the substituent Z having a large steric hindrance, which inhibits the association of molecules.

The sizes of the substituent Z and the carboxyl group (i.e., the numerals m and n) may be preferably larger for inhibiting the association of molecules by the steric hindrance, but when the sizes thereof are too large, the charge transfer from the molecules of the compound to the semiconductor may be inhibited, and thus the parameters are preferably in the aforementioned ranges.

The compound (A) may be produced, for example, by the known method disclosed in JP-A-2007-220412. For example, 5-tert-butyl-3,3-dibutyl-2-((E)-2-((E)-3-((E)-2-(5-tert-butyl-3,3-dibutyl-1-(2-carboxyethyl)indolin-2-ylidene)ethylidene)-2-chloro-5-ethylcyclohex-1-enyl)vinyl)-1-(2-carboxyethyl)-3H-indolium iodide represented by the following formula (a) (which may be hereinafter referred to as a compound A) may be obtained by reacting 1 mol of 2-chloro-5-ethyl-l-formyl-3-hydroxymethylenecyclohexene represented by the following formula (2) with 2 mol of 5-tert-butyl-3,3-dibutyl-1-carboxyethyl-2-methylindolenium iodide represented by the formula (3).

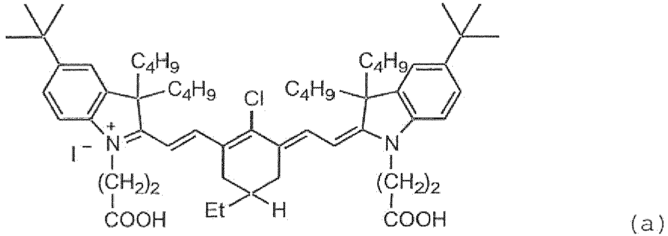

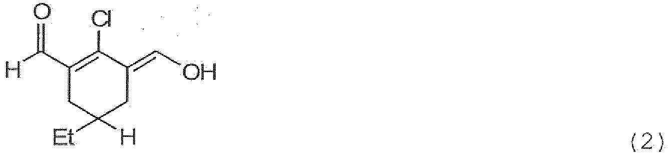

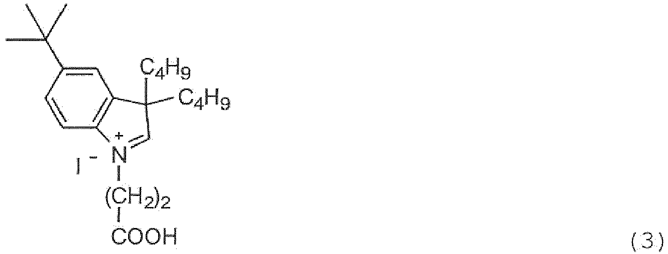

It is considered that the compound (B) of the invention contains the alkyl groups represented by R³ to R⁶ having a large number of carbon atoms and a large steric hindrance, which inhibits the association of molecules due to π-π stacking of the heptamethine structure and brings the advantageous effects as described above.

The compound (B) may also be produced, for example, by the known method disclosed in JP-A-2007-220412. For example, 1-(2-carboxyethyl)-2-((E)-2-((E)-3-((E)-2-(1-(2-carboxyethyl)-3,3-dioctylindolin-2-ylidene)ethylidene)-2-chloro-5-ethylcyclohex-1-enyl)vinyl)-3,3-dioctyl-3H-indolium iodide represented by the following formula (e) (which may be hereinafter referred to as a compound B) may be obtained by reacting 1 mol of 2-chloro-5-ethyl-1-formyl-3-hydroxymethylenecyclohexene represented by the following formula (4) with 2 mol of 3,3-dioctyl-1-carboxyethyl-2-methylindolenium iodide represented by the formula (5).

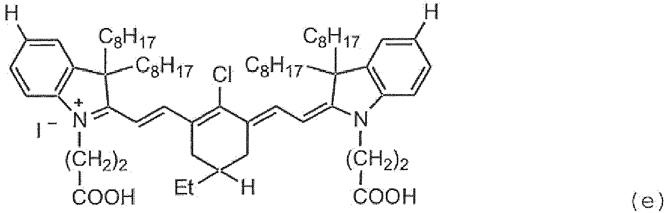

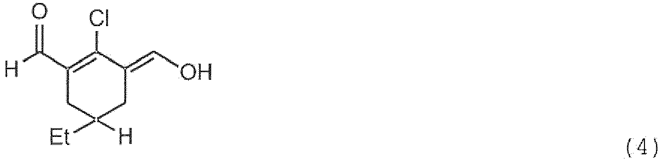

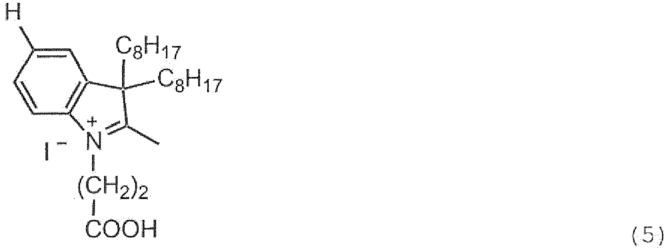

### 2. Photoelectric Conversion Device

The photoelectric conversion device of the invention contains the compound represented by the general formula (1) as a sensitizing dye. An embodiment for carrying out the photoelectric conversion device of the invention will be described below with reference to the attached drawing.

Fig. 1 is a schematic cross sectional view showing an example of a photoelectric conversion device 10 according to one embodiment of the invention.

In Fig. 1, numeral 1 denotes a transparent substrate, 2 denotes a transparent conductive film, 3 denotes a porous metal oxide semiconductor layer, 4 denotes a sensitizing dye, 5 denotes an electrolyte layer, 6 denotes a catalyst layer, 7 denotes an electrode substrate that holds the catalyst layer 6, 8 denotes an electrode substrate, and 9 denotes a counter electrode.

As shown in the figure, the porous metal oxide semiconductor layer 3 is formed on the surface of the electrode substrate 8 formed of the transparent substrate 1 and the transparent conductive film 2 formed thereon, and the sensitizing dye 4 is adsorbed on the surface of the porous metal oxide semiconductor layer 3. The counter electrode 9 having the catalyst layer 6 formed on the surface of the electrode substrate 7 is disposed with the electrolyte layer 5 of the invention intervening between them, thereby forming the photoelectric conversion device 10.

Preferred embodiments of the constitutional materials of the photoelectric conversion device 10 will be described below.

### Transparent Substrate

The transparent substrate 1 constituting the electrode substrate 8 may be a material that transmits visible light, and transparent glass is preferably used. Glass having a surface that has been treated to scatter incident light may also be used. In addition to glass, materials that transmit light, such as a plastic plate and a plastic film, may be used.

The thickness of the transparent substrate 1 is not particularly limited since it varies depending on the shape and the use conditions of the photoelectric conversion device 10. For example, in the case where glass or plastics are used, the thickness is preferably approximately from 1 mm to 1 cm in consideration of the durability in practical use. In the case where a plastic film or the like is used for providing flexibility, the thickness is preferably approximately from 1 µm to 1 mm.

### Transparent Conductive Film

The transparent conductive film 2 may be a material that transmits visible light and has conductivity. Examples of the material include a metal oxide. While not limited, tin oxide doped with fluorine (which may be hereinafter abbreviated as FTO), indium oxide, a mixture of tin oxide and indium oxide (which may be hereinafter abbreviated as ITO), tin oxide doped with antimony, zinc oxide, and the like may be preferably used.

An opaque conductive material may be used as far as it transmits visible light through such treatment as dispersion. Examples of the material include a carbonaceous material and a metal. Examples of the carbonaceous material include, while not limited, graphite, carbon black, glassy carbon, carbon nanotubes and fullerenes. Examples of the metal include, while not limited, platinum, gold, silver, ruthenium, copper, aluminum, nickel, cobalt, chromium, iron, molybdenum, titanium, tantalum, and alloys thereof.

Accordingly, the electrode substrate 8 may be formed by providing a conductive material containing at least one of the aforementioned conductive materials on the surface of the transparent substrate 1. In alternative, the electrode substrate 8 may also be formed by introducing the conductive material into the material constituting the transparent substrate 1, thereby integrating the transparent substrate 1 and the transparent conductive film 2.

Examples of the method of forming the transparent conductive film 2 using the metal oxide on the transparent substrate 1 include a liquid phase method, such as a sol-gel method, a gas phase method, such as sputtering and CVD, and a coating method of a dispersed paste. Examples of the method using the opaque conductive material include a method of binding the material in the form of powder or the like to the substrate with a transparent binder.

Examples of the method of integrating the transparent substrate 1 and the transparent conductive film 2 include a method of mixing the material for the conductive film as a conductive filler on molding the transparent substrate 1.

The thickness of the transparent conductive film 2 is not particularly limited since the conductivity varies depending on the material used, and the thickness for glass having an FTO film, which is generally used, may be from 0.01 to 5 µm, and preferably from 0.1 to 1 µm. The conductivity that is necessary therefor varies depending on the area of the electrode used, i.e., the resistance is preferably lower for a larger electrode, generally 100 Ω per square or less, preferably 10 Ω per square or less, and more preferably 5 Ω per square or less.

The thickness of the electrode substrate 8 formed of the transparent substrate 1 and the transparent conductive film 2 or the electrode substrate 8 having the transparent substrate 1 and the transparent conductive film 2 integrated is not particularly limited since it varies depending on the shape and the use conditions of the photoelectric conversion device 10, and is generally approximately from 1 µm to 1 cm.

### Porous Metal Oxide Semiconductor

The porous metal oxide semiconductor 3 is not particularly limited, examples of which include titanium oxide, zinc oxide and tin oxide, and in particular, titanium dioxide is preferred, with anatase-type titanium dioxide being more preferred.

The metal oxide preferably has less grain boundaries for decreasing the electric resistance. The semiconductor layer preferably has a large specific surface area for adsorbing a larger amount of the sensitizing dye, and specifically, the specific surface area thereof is preferably from 10 to 200 m²/g. Furthermore, for increasing the light absorption amount of the sensitizing dye, it is preferred that the oxide has varying particle diameters within a certain range for scattering light, and large oxide semiconductor particles having a particle diameter of from 300 to 400 µm are provided as a reflective layer on the porous layer.

The method of forming the porous metal oxide semiconductor layer 3 is not particularly limited, and the layer may be formed on the transparent conductive layer 2 by any known method. Examples of the method include a sol-gel method, a coating method of a dispersed paste, and a method of electrocrystallization or electrodeposition.

The thickness of the semiconductor layer 3 is not particularly limited since the optimum value thereof varies depending on the oxide used, and is generally from 0.1 to 50 µm, and preferably from 3 to 30 µm.

### Sensitizing Dye

The compound represented by the general formula (1) is used as the sensitizing dye 4. The compound may be used solely or as a mixture of two or more kinds thereof.

An additional sensitizing dye may also be used in combination in such a range that does not deviate the scope of the invention. While not limited, preferred examples of the additional sensitizing dye include a ruthenium complex, such as a ruthenium polypyridine complex, and examples of the additional sensitizing dye also include a metal complex dye other than the ruthenium complex, such as an iron complex and a copper complex, and further include an organic dye, such as a cyan dye, a porphyrin dye, a polyene dye, a coumarin dye, a cyanine dye, a squaric acid dye, a styryl dye, an eosin dye and an indoline dye. The dye preferably has a bonding group capable of bonding to the metal oxide semiconductor 3 for enhancing the electron injection efficiency to the metal oxide semiconductor 3. The bonding group is not particularly limited, and preferred examples thereof include a carboxyl group, a sulfonic acid group and a hydroxyl group.

Examples of the solvent for dissolving the sensitizing dye include an alcohol compound, such as ethanol, a nitrogen compound, such as acetonitrile, a ketone compound, such as acetone, an ether compound, such as diethyl ether, a halogenated aliphatic hydrocarbon compound, such as chloroform, an aliphatic hydrocarbon compound, such as hexane, an aromatic hydrocarbon compound, such as benzene, and an ester compound, such as ethyl acetate. The concentration of the dye in the solution may be appropriately controlled depending on the kinds of the dye and the solvent used, and a relatively higher concentration is preferred for adsorbing the dye to the surface of the semiconductor sufficiently. The concentration of the dye is preferably, for example, 4 x 10⁻⁵ mol/L or more.

The method of adsorbing the sensitizing dye 4 to the porous metal oxide semiconductor 3 is not particularly limited, and examples thereof include a method of immersing the electrode substrate 8 having formed thereon the porous metal oxide semiconductor 3 into a solution having the dye dissolved therein under conditions of room temperature and atmospheric pressure. The period of time of immersion may be preferably controlled appropriately depending on the kinds of the semiconductor, the dye and the solvent used and the concentration of the dye, thereby forming a monomolecular film of the sensitizing dye 4 uniformly on the semiconductor layer 3. The adsorption of the dye may be efficiently performed by immersion under heating. Deoxycholic acid, chenodeoxycholic acid or the like may be added as a co-adsorbing agent to the solution having the dye dissolved therein depending on the kind of the dye, and thereby the association of the sensitizing dye may be suppressed on adsorption on the semiconductor layer, and thus the photoelectric conversion efficiency of the solar cell device may be further enhanced.

### Electrolyte Layer

The electrolyte layer 5 may contain a supporting electrolyte, a redox pair capable of reducing the oxidized sensitizing dye, and a solvent capable of dissolving them. The redox pair constituting the electrolyte layer 5 may be any one that may be ordinarily used in a cell and a solar cell without particular limitation, examples of which include a combination of a diatomic molecule of halogen and a halide salt, a combination of thiocyanate anion and two molecules of thiocyanic acid, and an organic redox pair, such as a polypyridylcobalt complex and hydroquinone, and among these, a combination of an iodide molecule and an iodide is preferred.

As different from the case where iodine is used, for example, the use of a redox pair that has no strong absorption in the visible region and the infrared region not only facilitates effective utilization of the received light, but also enables production of a colorless and transparent photoelectric conversion device or a photoelectric conversion device having arbitrary color, which may enhance the applications of a solar cell. Examples of such a redox pair include a redox pair formed of a compound represented by the following formula (6) and a compound represented by the following formula (7):

RS⁻A⁺ (6)

RS-SR (7)

In the formulae (6) and (7), R represents a thiadiazole, triazole or tetrazole compound, and A represents an alkali metal atom or an onium compound.

A photoelectric conversion device that uses the redox pair having no strong absorption in the visible region and the infrared region has a lower photoelectric conversion capability than a photoelectric conversion device that uses an ordinary iodine redox pair, but shows an enhanced capability by using the compound represented by the general formula (1) of the invention as a sensitizing dye, as compared to the case where an ordinary sensitizing dye is used.

The solvent used in the electrolyte is not particularly limited as far as it is capable of dissolving the redox pair, and may be arbitrarily selected from a non-aqueous organic solvent, an ambient temperature molten salt, water, a protonic organic solvent and the like. Examples of the organic solvent include a nitrile compound, such as acetonitrile, methoxyacetonitrile, valeronitrile and 3-methoxypropionitrile, a lactone compound, such as γ-butyrolactone and valerolactone, a carbonate compound, such as ethylene carbonate and propylene carbonate, an ether compound, such as dioxane, diethyl ether and ethylene glycol dialkyl ether, an alcohol compound, such as methanol and ethanol, dimethylformamide, and an imidazole compound, and preferred examples thereof among these include acetonitrile, valeronitrile, 3-methoxypropionitrile, propylene carbonate and γ-butyrolactone. The solvent may be used solely or as a mixture of two or more kinds thereof.

The solvent used may be an ionic liquid, i.e., a molten salt. The ionic liquid is not particularly limited as far as it may be generally used in known cell and solar cell. Preferred examples of the cation of the molten salt include ammonium, imidazolium, oxazolium, thiazolium, oxadiazolium, triazolium, pyrrolidinium, pyridinium, piperidinium, pyrazolium, pyrimidinium, pyrazinium, triazinium, phosphonium, sulfonium, carbazolium, indolium, and derivatives thereof, and ammonium, imidazolium, pyridinium, piperidinium, pyrazolium and sulfonium are particularly preferred. Examples of the anion of the molten salt include a metal chloride, such as AlCl₄⁻ and Al₂Cl₇⁻, a fluorine-containing compound, such as PF₆⁻, BF4⁻, CF₃SO₃⁻, N(CF₃SO₂)₂⁻, N(F₂SO₂)₂⁻, F(HF)ₙ⁻ and CF₃COO⁻, a non-fluorine compound, such as NO₃⁻, CH₃COO⁻, C₆H₁₁COO⁻, CH₃OSO₃⁻, CH₃OS₂⁻, CH₃SO₃⁻, CH₃SO₂⁻, (CH₃O)₂PO₂⁻, SCN⁻, N(CN)₂⁻ and B(CN)₄⁻, and a halide, such as an iodide and a bromide.

The concentration of the redox pair in the electrolyte layer (solvent) may vary depending on the viscosity of the solvent used and is preferably in a range of from 0.01 to 1.2 mol/L. In the case where the preferred solvent, such as acetonitrile, valeronitrile, 3-methoxypropionitrile, propion carbonate and γ-butyrolactone, is used, the concentration of the redox pair is particularly preferably in a range of from 0.02 to 0.6 mol/L. When the concentration of the redox pair is too low, the charge transfer capability may be insufficient and deteriorate the capability of the device, and when the concentration of the redox pair is too high, reverse electron transfer from the dye or the semiconductor layer to the redox pair may occur to deteriorate the capability of the device.

The electrolyte layer 5 may contain a supporting electrolyte, an additive and the like in such a range that does not deviate from the objects of the invention and does not impair the characteristics of the electrolyte layer. Examples of the supporting electrolyte include a lithium salt, an imidazolium salt and a quaternary ammonium salt. Examples of the additive include a base, such as t-butylpyridine, N-methylimidazole, N-methylbenzimidazole and N-methylpyrrolidone, and a thiocyanate compound, such as guaniduim thiocyanate. The electrolyte layer may be physically or chemically gelled by adding a suitable gelling agent.

### Counter Electrode

The counter electrode 9 has a structure that contains an electrode substrate 7 having formed on the surface thereof a catalyst layer 6. The electrode substrate 7 preferably has conductivity on the surface portion thereof since it is used as both a support and a collector for the catalyst layer 6.

Preferred examples of the material therefor include a metal, a metal oxide, a carbonaceous material and a conductive polymer, which have conductivity. Examples of the metal include platinum, gold, silver, ruthenium, copper, aluminum, nickel, cobalt, chromium, iron, molybdenum, titanium, tantalum, and alloys thereof. Examples of the carbonaceous material include, while not limited, graphite, carbon black, glassy carbon, carbon nanotubes and fullerenes. In the case where a metal oxide, such as FTO, ITO, indium oxide, zinc oxide and antimony oxide, is used, the amount of incident light on the sensitizing dye layer 4 may be increased since these materials are transparent or translucent.

An insulating material, such as glass and plastics, may be used as the electrode substrate 7 if it is treated to impart conductivity at least to the surface portion thereof. Examples of the method for imparting conductivity to such an insulating material include a method of covering a part or the whole surface of the insulating material with the aforementioned conductive material, for which a solution method, such as plating and electrodeposition, and a gas phase method, such as sputtering and vacuum vapor deposition, may be used for the case where a metal is used, and a sol-gel method or the like may be used for the case where a metal oxide is used. Examples of the method also include a method of mixing one kind or plural kinds of powder of the conductive material with the insulating material.

Even in the case where an insulating material is used as the electrode substrate 7 of the counter electrode 9, the catalyst layer 6 that has high conductivity may be formed on the substrate 7, and thereby the assembly may be used as the counter electrode 9 since the catalyst layer 6 can solely function as both a collector and a catalyst.

The shape of the electrode substrate 7 is not particularly limited and may vary depending on the shape of the photoelectric conversion device 10, in which the catalyst electrode is used, and may be, for example, a plate shape or a film shape having flexibility. The electrode substrate 7 may be either transparent or opaque, and is preferably transparent or translucent since the amount of incident light on the sensitizing dye layer 4 may be increased, and improved design may be imparted in some cases.

The electrode substrate 7 used may be generally a glass plate having an FTO film, a PET film having an ITO film, a PEN film having an ITO film, or the like, and the thickness of the conductive layer is not particularly limited since the conductivity varies depending on the material used. For example, the thickness of the conductive layer may be from 0.01 to 5 µm, and preferably from 0.1 to 1 µm, for a glass plate having an FTO film.

The conductivity that is necessary therefor may vary depending on the area of the electrode used, and a lower resistance may be demanded for the electrode with a larger area. The resistance is generally 100 Ω per square or less, preferably 10 Ω per square or less, and more preferably 5 Q per square or less.

The thickness of the electrode substrate 7 is not particularly limited since it may vary depending on the shape and the use conditions of the photoelectric conversion device 10, and is generally approximately 1 µm to 1 cm.

The catalyst layer 6 is not particularly limited as far as it has such an electrode property that can rapidly perform the reaction of reducing the oxidant of the redox pair in the electrolyte to the reductant. Examples thereof include a platinum catalyst electrode, such as an electrode having chloroplatinic acid coated and heat-treated thereon and an electrode having platinum vapor-deposited thereon, a carbonaceous material, such as activated carbon, glassy carbon and carbon nanotubes, an inorganic sulfur compound, such as cobalt sulfide, and a conductive polymer, such as polythiophene, polypyrrole and polyaniline, and among these, a conductive polymer catalyst is preferably used.

Preferred examples of the monomer constituting the conductive polymer catalyst include a thiophene compound. Preferred examples of the thiophene compound include thiophene, tetradecylthiophene, isothianaphthene, 3-phenylthiophene, 3,4-ethylenedioxythiophene, and derivatives thereof, and among these, 3,4-ethylenedioxythiophene and a derivative thereof are preferably used. Examples of the derivative of 3,4-ethylenedioxythiophene include hydroxymethyl-3,4-ethylenedioxythiophene, aminomethyl-3,4-ethylenedioxythiophene, hexyl-3,4-ethylenedioxythiophene and octyl-3,4-ethylenedioxythiophene. The thiophene compound may be used solely or as a combination of two or more kinds thereof for forming the conductive polymer catalyst layer 6.

The monomer used for forming the conductive polymer catalyst layer 6 preferably exhibits a conductivity of 10⁻⁹ S/cm or more in the form of a polymerized film.

The conductive polymer catalyst layer 6 preferably contains a dopant for enhancing the conductivity. The dopant used may be a known material without particular limitation.

Specific examples of the dopant include a halogen anion, such as iodide, bromide and chloride, a halide anion, such as hexafluorophosphorus, hexafluoroarsenic, hexafluoroantimony, tetrafluoroboron and perchloric acid, an alkyl group-substituted organic sulfonate anion, such as methanesulfonic acid and dodecylsulfonic acid, a cyclic sulfonate anion, such as camphorsulfonic acid, an alkyl group-substituted or unsubstituted benzene mono- or disulfonate anion, such as benzenesulfonic acid, p-toluenesulfonic acid, dodecylbenzenesulfonic acid and benzenedisulfonic acid, an alkyl group-substituted or unsubstituted naphthalenesulfonate anion having from 1 to 3 sulfonic acid group(s) substituted thereon, such as 2-naphthalenesulfonic acid and 1,7-naphthalenedisulfonic acid, an alkyl group-substituted or unsubstituted biphenylsulfonate anion, such as anthracenesulfonic acid, anthraquinonesulfonic acid, alkylbiphenylsulfonic acid and biphenyldisulfonic acid, a polymer sulfonate anion, such as polystyrenesulfonic acid and a naphthalenesulfonic acid-formalin condensate, a substituted or unsubstituted aromatic sulfonate anion, a boron compound anion, such as boron bissalicylate and boron biscatecholate, a heteropoly acid anion, such as molybdophosphoric acid, tungstophosphoric acid and tungstomolybdophosphoric acid, and an imidic acid. The dopant may be used solely or as a combination of two or more kinds thereof.

For desorption of the dopant, an organic acid anion is preferred than an inorganic anion, and an anion that is difficult to suffer thermal decomposition is preferred. A dopant in the form of a low molecular weight compound is preferred than a dopant of a polymer compound since a dopant in the form of a low molecular weight compound has a higher catalyst activity for the redox pair in the invention. Specific examples thereof include p-toluenesulfonic acid, dodecylbenzenesulfonic acid and naphthalenesulfonic acid.

The amount of the dopant used in the conductive polymer catalyst layer is not particularly limited since the optimum value thereof may vary depending on the kind of the dopant used, and is preferably from 5 to 60% by mass, and more preferably from 10 to 45% by mass.

The dopant may be used in combination with the monomer of the conductive polymer on forming the conductive polymer catalyst layer.

The conductive polymer catalyst layer 6 is formed on the electrode substrate 7. The method of forming the catalyst layer is not particularly limited, and examples thereof include a method of forming a film from the conductive polymer in a molten state or a solution having the conductive polymer dissolved therein.

The catalyst layer preferably has a porous state with a large surface area, and therefore, for example, such a method may be preferably used that the monomer of the conductive polymer is oxidatively polymerized chemically or electrochemically in a state where the solution containing the monomer is in contact with the electrode substrate 7.

Such a method may also be used that powder of the conductive polymer is formed into a paste, an emulsion or a mixture containing the polymer solution and a binder, which is then formed into the catalyst layer on the electrode substrate 7 by screen printing, spray coating, brush coating or the like.

Among the methods for forming the conductive polymer catalyst layer 6 described above, the electrolytic polymerization method and the chemical polymerization method are preferred, and the chemical polymerization method is more preferred. The chemical polymerization method is a method for oxidatively polymerizing a polymerizable monomer with an oxidizing agent. The electrolytic polymerization method is a method of forming a film of a conductive polymer on an electrode, such as a metal, by performing electrolytic oxidation in a solution containing a polymerizable monomer.

Examples of the oxidizing agent used in the chemical polymerization method include a halide, such as iodine, bromine, bromine iodide, chlorine dioxide, iodic acid, periodic acid and chlorous acid, a metal halide, such as antimony pentafluoride, phosphorus pentachloride, phosphorus pentafluoride, aluminum chloride and molybdenum chloride, a high valent metal salt, such as a permanganate salt, a dichromate salt, chromic anhydride, ferric salt and cupric salt, a protonic acid, such as sulfuric acid, nitric acid and trifluoromethanesulfuric acid, an oxygen compound, such as sulfur trioxide and nitrogen dioxide, a peroxo acid and a salt thereof, such as hydrogen peroxide, ammonium persulfate and sodium perborate, and a heteropoly acid and a salt thereof, such as molybdophosphoric acid, tungstophosphoric acid and tungstomolybdophosphoric acid, and at least one selected therefrom may be used.

Although the chemical polymerization method is suitable for mass production, the polymer obtained by reacting the aromatic compound monomer in the solution with the oxidizing agent is in the form of particles or bulk, and it is difficult to form the polymer into the desired porous material in the form of the electrode. Accordingly, the conductive polymer is formed preferably in such a manner that the electrode substrate 7 is immersed in a solution containing any one of the aromatic compound monomer and the oxidizing agent or coated with the solution, and subsequently the electrode substrate is immersed in a solution containing the other component or coated with the solution, thereby performing the polymerization on the surface of the electrode substrate 7.

In alternative, the porous conductive polymer film may also be formed of such a manner that an additive that reduces the polymerization rate is added to a solution containing the monomer and a polymerization initiator, and after forming the solution into a film under a non-polymerizing condition at room temperature, the film is heated for performing the polymerization reaction. The method for forming the film is not particularly limited, and examples thereof include a spin coating method, a casting method, a squeegee coating method and a screen printing method.

As for the additive that reduces the polymerization rate, the addition of a base may reduce the polymerization rate in the case where the polymerization initiator is a high valent metal salt, such as an Fe(III) salt. This is because the oxidation potential of the Fe(III) salt varies by pH, as described in Synthetic Metals, vol. 66, p. 263(1994). Examples of the base include imidazole and dimethylsulfoxide.

The solvent for mixing and dissolving the monomer, the polymerization initiator and the additive is not particularly limited as far as it dissolves the compounds used but does not dissolve the electrode substrate 7 and the polymerized product, and examples thereof include an alcohol compound, such as methanol, ethanol, propanol and n-butanol.

While the mixing ratio of the monomer, the polymerization initiator and the additive may vary depending on the compounds used and the target polymerization degree and polymerization rate, the molar ratio of the polymerization initiator to the monomer, i.e., monomer/polymerization initiator, is preferably from 1/0.3 to 1/10, and the molar ratio of the additive to the polymerization initiator, i.e., polymerization initiator/additive, is preferably from 1/0.05 to 1/4.

While the heating condition for heat polymerization after coating the solution may vary depending on the kinds of the monomer, the polymerization catalyst and the additive, the mixing ratio and the concentrations thereof, and the thickness of the coated film, the heating condition preferably includes a heating temperature of from 25 to 120°C in the air and a heating time of from 1 minute to 12 hours.

In alternative, furthermore, the porous conductive polymer film may also be formed by such a method that the conductive polymer film is formed on the surface of the electrode substrate 7 or the electrode substrate having a conductive film by using a dispersion or a paste of the conductive polymer particles having been prepared in advance, and then the aforementioned chemical polymerization is performed to grow conductive polymer particles thereon.

The thickness of the catalyst layer 6 in the counter electrode 9 is preferably from 5 nm to 5 µm, and particularly preferably from 50 nm to 2 µm.

After preparing the constitutional members as described above, the metal oxide semiconductor electrode and the catalyst electrode are assembled to face each other with the electrolyte intervening between them, and thus the photoelectric conversion device 10 is completed.

### EXAMPLE

The invention will be described in more detail with reference to examples below, but the invention is not limited to the examples.

### 1. Synthesis of Compound represented by General Formula (1)

### (1) Synthesis of Compound (A)

### Synthesis of Compound (a)

The compound (a) represented by the following formula (5-tert-butyl-3,3-dibutyl-2-((E)-2-((E)-3-((E)-2-(5-tert-butyl-3,3-dibutyl-1-(2-carboxyethyl)indolin-2-ylidene)ethylidene)-2-chloro-5-ethylcyclohex-1-enyl)vinyl)-1-(2-carboxyethyl)-3H-indolium iodide) was synthesized in the following manner.

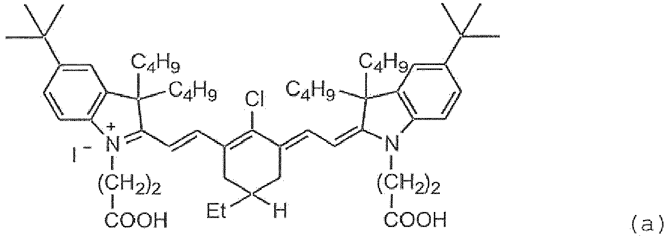

2-Chloro-5-ethyl-1-formyl-3-hydroxymethylenecyclohexene (0.217 g, 1.08 mmol) and 5-tert-butyl-3,3-dibutyl-1-carboxyethyl-2-methylindolenium iodide (1.002 g, 2.01 mmol) were reacted in N,N-dimethylformamide (hereinafter referred to as DMF) (5 mL) at 130°C for 2 hours. After completing the reaction, the reaction product was added to distilled water (100 mL), and the solid matter thus deposited was recovered, extracted three times with ethyl acetate (20 mL x 3), and then dehydrated with anhydrous sodium sulfate. After removing the solvent, the target substance was obtained through purification by silica gel column chromatography (dichloromethane/methanol = 20/1) with a yield of 48% (0.498 g). The resulting substance was measured for the Rf value of thin layer chromatography, the melting point, the infrared spectroscopy (IR), the nuclear magnetic resonance (¹H-NMR and ¹³C-NMR) and the high resolution mass spectroscopy (HRMS). The results obtained are shown below.

Rf: 0.18 (dichloromethane/methanol = 10/1)
melting point: 161.5 - 162.3°C
IR (KBr): 1,732 (C=O), 2,932 (O-H) cm⁻¹
¹H-NMR (CD₃OD) : δ 0.49-0.58 (m, 4H), 0.71 (t, J = 7.24 Hz, 6H), 0.74 (t, J = 7.24 Hz, 6H), 0.83-0.98 (m, 4H), 1.09-1.21 (m, 11H), 13.4 (s, 18H), 1.57 (quint, J = 7.24 Hz, 2H), 1.80 (s, 1H), 2.25-2.29 (m, 10H), 2.79 (t, J = 6.52 Hz, 4H), 2.99-3.03 (m, 2H), 4.48 (t, J = 6.52 Hz, 4H), 6.54 (d, J = 14.01 Hz, 2H), 7.29 (d, J = 8.45 Hz, 2H), 7.48-7.51 (m, 4H), 8.43 (d, J = 14.01 Hz, 2H)
¹³C-NMR (CD₃OD): δ 12.0, 14.0, 23.5, 23.5, 27.1, 29.4, 31.8, 31.8, 32.9, 33.8, 35.2, 35.9, 41.8, 42.8, 60.3, 103.7, 111.5, 120.5, 126.8, 127.7, 139.4, 142.8, 144.4, 150.5, 171.6
HRMS (FAB): m/z calcd for C₅₈H₈₄Cl₁N₂O₄: 907.6114 (M-I), found: 907.6118

### Synthesis of Compound (b)

The compound (b) represented by the following formula (3,3-dibutyl-1-(2-carboxyethyl)-2-((E)-2-((E)-2-chloro-3-((E)-2-(3,3-dibutyl-1-(2-carboxyethyl)-5-methoxyindolin-2-ylidene)ethylidene)-5-ethylcyclohex-1-enyl)vinyl)-5-methoxy-3H-indolium iodide) was synthesized in the following manner.

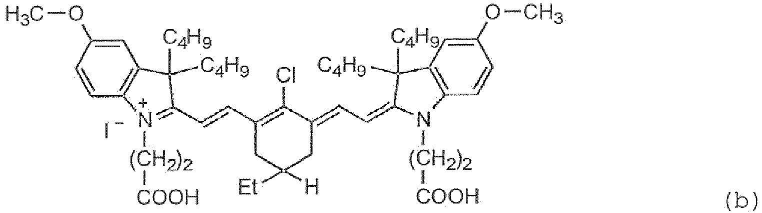

2-Chloro-5-ethyl-1-formyl-3-hydroxymethylenecyclohexene (1.061 g, 5.29 mmol) and 3,3-dibutyl-1-carboxyethyl-5-methoxy-2-methylindolenium iodide (3.784 g, 7.99 mmol) were reacted in DMF (10 mL) at 130°C for 2 hours. After completing the reaction, the reaction product was added to distilled water (100 mL), and the solid matter thus deposited was recovered, extracted three times with ethyl acetate (20 mL x 3), and then dehydrated with anhydrous sodium sulfate. After removing the solvent, the target substance was obtained through purification by silica gel column chromatography (dichloromethane/methanol = 20/1) with a yield of 41% (1.614 g). The resulting substance was measured for the Rf value of thin layer chromatography, the melting point, IR, ¹H-NMR, ¹³C-NMR and HRMS. The results obtained are shown below.

Rf: 0.35 (dichloromethane/methanol = 10/1)
melting point: 128.5 - 129.7°C
IR (KBr) : 1,732 (C=O), 3,021 (O-H) cm⁻¹
¹H-NMR (CD₃OD) : δ 0.54-0.60 (m, 4H), 0.74 (t, J = 7.24 Hz, 6H), 0.75 (t, J = 7.24 Hz, 6H), 0.84-0.90 (m, 4H), 1.09-1.20 (m, 11H), 1.57 (quint, J = 7.24 Hz, 2H), 1.79 (s, 1H), 2.15-2.31 (m, 10H), 2.81 (t, J = 6.52 Hz, 4H), 2.89-3.01 (m, 2H), 3.30-3.32 (m, 2H), 3.86 (s, 6H), 4.45 (s, 4H), 7.01 (dd, J = 8.69, 2.42 Hz, 2H), 7.07 (d, J = 2.42 Hz, 2H), 7.28 (d, J = 8.69 Hz, 2H), 8.36 (d, J = 13.04 Hz, 2H)
¹³C-NMR (CD₃OD) : δ 12.1, 14.1, 14.1, 23.5, 23.6, 27.0, 29.3, 33.1, 35.1, 41.6, 42.9, 55.0, 56.6, 60.1, 103.7, 107.0, 112.8, 114.7, 127.3, 138.2, 141.2, 143.3, 149.6, 160.0, 170.4, 173.5
HRMS (FAB): m/z calcd for C₅₂H₇₂Cl₁N₂O₆: 855.5073 (M-I), found: 855.5078

### Synthesis of Compound (c)

The compound (c) represented by the following formula (3,3-dibutyl-1-(2-carboxyethyl)-2-((E)-2-((E)-2-chloro-3-((E)-2-(3,3-dibutyl-1-(2-carboxyethyl)-5-isopropylindolin-2-ylidene)ethylidene)-5-ethylcyclohex-1-enyl)vinyl)-5-isopropyl-3H-indolium iodide) was synthesized in the following manner.

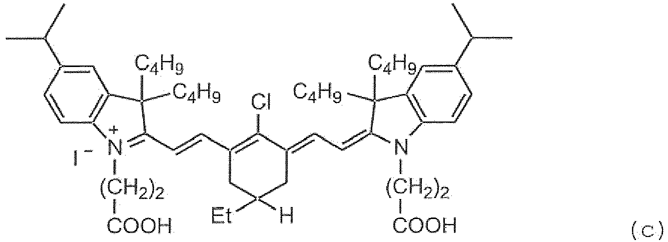

2-Chloro-5-ethyl-1-formyl-3-hydroxymethylenecyclohexene (0.610 g, 3.04 mmol) and 3,3-dibutyl-1-carboxyethyl-5-isopropyl-2-methylindolenium iodide (2.874 g, 5.92 mmol) were reacted in DMF (10 mL) at 130°C for 2 hours. After completing the reaction, the reaction product was added to distilled water (100 mL), and the solid matter thus deposited was recovered, extracted three times with ethyl acetate (20 mL x 3), and then dehydrated with anhydrous sodium sulfate. After removing the solvent, the target substance was obtained through purification by silica gel column chromatography (dichloromethane/methanol = 20/1) with a yield of 38% (1.145 g). The resulting substance was measured for the Rf value of thin layer chromatography, the melting point, IR, ¹H-NMR, ¹³C-NMR and HRMS. The results obtained are shown below.

Rf: 0.35 (dichloromethane/methanol = 10/1)
melting point: 120.5 - 121.2°C
IR (KBr) : 1,732 (C=O), 2,932 (O-H) cm⁻¹
¹H-NMR (CD₃OD) : δ 0.37-0.48 (m, 4H), 0.61 (t, J = 7.24 Hz, 6H), 0.62 (t, J = 7.24 Hz, 6H), 0.74-0.85 (m, 4H), 0.99-1.11 (m, 11H), 1.17-1.14 (m, 13H), 1.45-1.51 (m, 2H), 1.69 (s, 1H), 2.13-2.21 (m, 10H), 2.73 (t, J = 6.28 Hz, 4H), 3.01 (m, 4H), 4.03 (s, 4H), 6.44 (d, J = 13.77 Hz, 2H), 7.21 (m, 6H), 8.30 (d, J = 13.77 Hz, 2H)
¹³C-NMR (CD₃OD): δ 12.0, 14.0, 23.5, 24.7, 27.0, 29.3, 32.9, 33.1, 35.1, 35.3, 41.5, 42.8, 60.1, 103.9, 111.9, 121.4, 127.8, 139.5, 142.9, 144.1, 148.1, 150.4, 171.4, 173.7 HRMS (FAB): m/z calcd for C₅₆H₈₀Cl₁N₂O₄: 879.5801 (M-I), found: 879.5806

### Synthesis of Compound (d)

The compound (d) represented by the following formula (5-tert-butyl-2-((E)-2-((E)-3-((E)-2-(5-tert-butyl-1-(2-carboxyethyl)-3-heptyl-3-octylindolin-2-ylidene)ethylidene)-2-chloro-5-ethylcyclohex-1-enyl)vinyl)-1-(2-carboxyethyl)-3,3-dioctyl-3H-indolium iodide) was synthesized in the following manner.

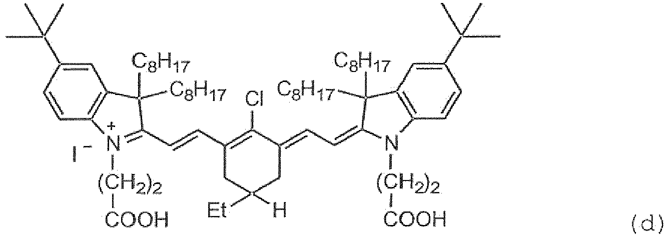

2-Chloro-5-ethyl-1-formyl-3-hydroxymethylenecyclohexene (0.199 g, 1.00 mmol) and 5-tert-butyl-1-carboxyethyl-2-methyl-3,3-dioctylindolenium iodide (1.306 g, 2.13 mmol) were reacted in DMF (5 mL) at 130°C for 2 hours. After completing the reaction, the reaction product was added to distilled water (100 mL), and the solid matter thus deposited was recovered, extracted three times with ethyl acetate (20 mL x 3), and then dehydrated with anhydrous sodium sulfate. After removing the solvent, the target substance was obtained through purification by silica gel column chromatography (dichloromethane/methanol = 20/1) with a yield of 38% (0.478 g). The resulting substance was measured for the Rf value of thin layer chromatography, the melting point, IR, ¹H-NMR, ¹³C-NMR and HRMS. The results obtained are shown below.

Rf: 0.34 (dichloromethane/methanol = 10/1) melting point: 112.5 - 113.4°C
¹H-NMR (CD₃OD) : δ 0.59 (br s, 4H), 0.81 (t, J = 6.73 Hz, 6H), 0.82 (t, J = 6.73 Hz, 6H), 0.94 (br s, 4H), 1.13-1.21 (m, 43H), 1.37 (s, 18H), 1.58-1.61 (m, 2H), 1.81 (br s, 1H), 2.22-2.34 (m, 10H), 2.63 (t, J = 13.00, 4H), 3.02 (d, J = 13.9, 2H), 4.42 (br s, 4H) 6.53 (d, J = 13.50 Hz, 2H), 7.36 (d, J = 8.10 Hz, 2H), 7.49 (t, J =8.10, 4H), 8.43 (d, J = 13.50 Hz, 2H)
¹³C-NMR (CD₃OD) : δ 10. 9, 13.6, 22.5, 23.1, 28.1, 28.5, 28.8, 29.0, 31.0, 31.3, 31.7, 34.0, 34.4, 35.1, 41.6, 41.8, 58.9, 102.0, 110.4, 119.0, 125.7, 126.0, 138.0, 141.6, 143.1, 149.0, 149.1, 170.0

### (2) Synthesis of Compound (B)

### Synthesis of Compound (e)

The compound (e) represented by the following formula (1-(2-carboxyethyl)-2-((E)-2-((E)-3-((E)-2-(1-(2-carboxyethyl)-3,3-dioctylindolin-2-ylidene)ethylidene)-2-chloro-5-ethylcyclohex-1-enyl)vinyl)-3,3-dioctyl-3H-indolium iodide) was synthesized in the following manner.

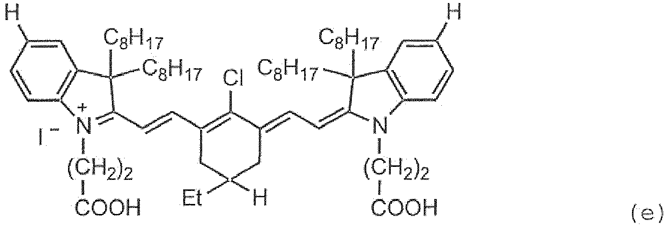

2-Chloro-5-ethyl-1-formyl-3-hydroxymethylenecyclohexene (0.361 g, 1.80 mmol) and 3,3-dioctyl-1-carboxyethyl-2-methylindolenium iodide (1.992 g, 3.60 mmol) were reacted in DMF (10 mL) at 120°C for 2.5 hours. After distilled water (50 mL) was added to the reaction product for terminating the reaction, and the reaction product was extracted with ethyl acetate, rinsed with saturated saline (30 mL) and water twice (30 mL x 2), and then dehydrated with anhydrous sodium sulfate. The target substance was obtained through purification by silica gel column chromatography (dichloromethane/methanol = 20/1) with a yield of 32% (0.670 g). The resulting substance was measured for the Rf value of thin layer chromatography, the melting point, the infrared spectroscopy (IR), the nuclear magnetic resonance (¹H-NMR and ¹³C-NMR) and the high resolution mass spectroscopy (HRMS). The results obtained are shown below.

Rf: 0.53 (dichloromethane/methanol = 10/1)
melting point: 57.0 - 58.5°C
IR (KBr): 1,732 (C=O), 2,928 (O-H) cm⁻¹
¹H-NMR (CD₃OD) : δ 0.56 (br, 4H), 0.80 (dt, J = 2.5, 6.9 Hz, 16H), 1.07-1.11 (m, 43H), 1.57 (quint., J = 7.1 Hz, 2H), 1.78 (br, 1H), 2.16-2.28 (m, 10H),2.69 (t, J = 7.0 Hz, 4H), 2.99-3.03 (m, 2H), 4.44 (t, J = 6.8 Hz, 4H), 6.56 (d, J = 14.0 Hz, 2H), 7.30 (t, J = 7.2 Hz, 2H), 7.38-7.46 (m, 6H), 8.43 (d, J = 14.0 Hz, 2H)
¹³C-NMR (CD₃OD) : δ 12.9, 15.5, 24.6, 25.3, 30.3, 30.7, 31.0, 31.2, 33.9, 36.1, 43.2, 43.9, 61.1, 104.6, 113.0, 124.2, 127.5, 128.8, 140.4, 145.8, 172.6, 179.3
HRMS (FAB): m/z calcd for C₆₆H₁₀₀ClN₂O₄: 1019.7366 (M-I), found: 1019.7362

### Synthesis of Compound (f)

The compound (f) represented by the following formula (1-(2-carboxyethyl)-2-((E)-2-((E)-3-((E)-2-(1-(2-carboxyethyl)-3,3-didodecylindolin-2-ylidene)ethylidene)-2-chloro-5-ethylcyclohex-1-enyl)vinyl)-3,3-didodecyl-3H-indolium iodide) was synthesized in the following manner.

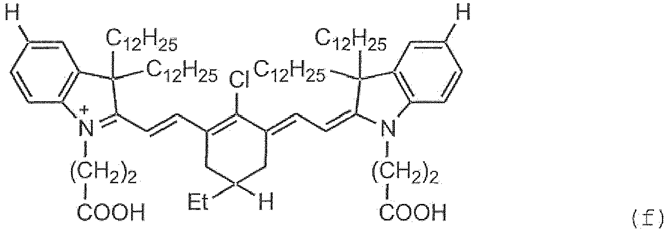

2-Chloro-5-ethyl-1-formyl-3-hydroxymethylenecyclohexene (0.200 g, 1.0 mmol) and 3,3-didodecyl-1-carboxyethyl-2-methylindolenium iodide (1.390 g, 2.0 mmol) were reacted in DMF (5 mL) at 120°C for 2.5 hours. After completing the reaction, the reaction product was extracted with ethyl acetate, rinsed with saturated saline (50 mL) and water twice (50 mL x 2), and then dehydrated with anhydrous sodium sulfate. The target substance was obtained through purification by silica gel column chromatography (dichloromethane/methanol = 20/1) with a yield of 25% (0.346 g). The resulting substance was measured for the Rf value of thin layer chromatography, the melting point, IR, ¹H-NMR, ¹³C-NMR and HRMS. The results obtained are shown below.

Rf: 0.5 (dichloromethane/methanol = 10/1)
melting point: < 30°C
IR (KBr): 1,732 (C=O), 2,924 (O-H) cm^{-1 1}H-NMR (CD₃OD): δ 0.58 (br, 4H), 0.88 (t, J = 6.8 Hz, 12H), 1.10-1.37 (m, 79H), 1.60 (quint., J = 7.2 Hz, 2H), 1.80 (br s, 1H), 2.18-2.34 (m, 10H),2.81 (t, J = 6.5 Hz, 4H), 3.04-3.07 (m, 2H), 4.51 (t, J = 6.3 Hz, 4H), 6.58 (d, J = 14.0 Hz, 2H), 7.32 (t, J = 7.2 Hz, 2H), 7.39-7.48 (m, 6H), 8.46 (d, J = 14.0 Hz, 2H)
¹³C-NMR (CD₃OD) : δ 12.0, 14.6, 23.8, 24.4, 29.8, 30.2, 30.4, 30.6, 30.8, 30.8, 30.8, 33.1, 41.6, 43.0, 60.2, 103.9, 112.2, 123.4, 126.7, 128.1, 130.1, 139.4, 144.8, 145.0, 151.3, 171.8
HRMS (FAB): m/z calcd for C₈₂H₁₃₂ClN₂O₄, 1-243. 9870 (M-I) ; found, 1243.9879

### (3) Synthesis of Comparative Dye

2-Chloro-5-ethyl-1-formyl-3-hydroxymethylenecyclohexene (0.40 g) and 3,3-dibutyl-1-carboxyethyl-2-methylindolenium iodide (1.79 g) were dissolved in acetic acid (100 mL) and reacted under refluxing for 3 hours. After cooling, water was added to the reaction solution, and the solid matter thus deposited was recovered by filtration. 0.61 g of the comparative dye represented by the following formula was obtained through purification by silica gel column chromatography (dichloromethane/methanol = 10/1).

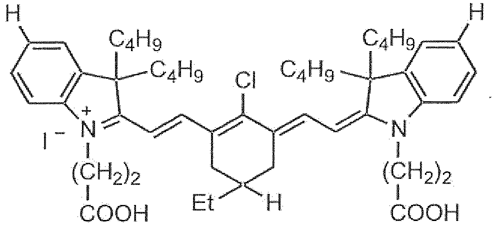

### 2. Production of Solar Cell

Solar cells were produced according to the following manner by using the compounds (a) to (f) as sensitizing dyes for examples and the comparative dye as a sensitizing dye for a comparative example, and evaluated for the capability.

### Production of Porous Metal Oxide Semiconductor

A transparent conductive film 2 formed of SnO₂ doped with fluorine was formed by vacuum vapor deposition on a transparent substrate 1 formed of glass, on which a porous metal oxide semiconductor layer 3 was formed by the following manner.

FTO glass (produced by Nippon Sheet Glass Co. Ltd.) was used as an electrode substrate 8 containing a substrate 1 having a transparent conductive film 2 formed thereon. On the surface of the electrode substrate on the side of the transparent conductive film 2, a commercially available titanium oxide paste (TSP-18NR, a trade name, produced by JGC Catalysts and Chemicals Ltd., particle size: 20 nm) was printed by a screen printing method to a thickness of approximately 6 µm over an area of approximately 5 mm x 10 mm, and further thereon, a commercially available titanium oxide paste (TSP-400C, a trade name, produced by JGC Catalysts and Chemicals Ltd., particle size: 400 nm) was applied by a screen printing method to a thickness of approximately 4 µm over the same area, followed by baking in the air at 500°C for 30 minutes. As a result, a titanium oxide film (i.e., a porous metal oxide semiconductor film 3) having a thickness of approximately 10 µm was obtained.

### Adsorption of Sensitizing Dye

Dye solutions containing 0.1 mM of either of the compounds (a) to (d) and the comparative dye synthesized above and 2 mM of deoxycholic acid as a co-absorbing agent dissolved in anhydrous ethanol as a solvent were prepared. The porous titanium oxide semiconductor electrode produced above was immersed in either of the dye solutions for 1 hour, and the excessive dye was washed out with anhydrous ethanol, followed by drying in the air, thereby producing a semiconductor electrode for a solar cell.

### Preparation of Electrolytic Solution

The following electrolytic solutions 1 to 3 were prepared as an electrolytic solution constituting an electrolytic layer 5.

### Electrolytic Solution 1

3-Methoxypropionitile was used as a solvent, in which 0.1 mol/L of 5,5'-dithiobis(1-methyl-1H-tetrazole), 0.05 mol/L of 1-methyl-5-mercapto-1,2,3,4-tetrazole lithium salt and 0.05 mol/L of 1-methyl-5-mercapto-1,2,3,4-tetrazole 1-methyl-3-ethylimidazolium salt (EMIm-TZT) were dissolved, thereby preparing the electrolytic solution 1.

### Electrolytic Solution 2

3-Methoxypropionitile was used as a solvent, in which 0.15 mol/L of 5-methyl-2-mercapto-1,3,4-thiadiazole 1-methyl-3-ethylimidazolium salt, 0.15 mol/L of 2,2'-dithiobis(5-methyl-1,3,4-thiadiazole) and 0.1 mol/L of tert-butylpyridine were dissolved, thereby preparing the electrolytic solution 2.

### Electrolytic Solution 3

3-Methoxypropionitile was used as a solvent, in which 0.6 mol/L of 1,2-dimethyl-3-propylimidazolium iodide, 0.1 mol/L of lithium iodide and 0.1 mol/L of iodine were dissolved, thereby preparing the electrolytic solution 3.

The sulfide redox materials used in the electrolytic solutions were synthesized in the following manners. The other materials than those described were commercially available products or materials that were prepared from commercially available products by known methods.

### Synthesis of 5,5'-Dithiobis(1-methyl-1H-tetrazole)

1 mol equivalent of 1-methyl-1,2,3,4-tetrazole-5-thiol and 0.5 mol equivalent of potassium carbonate was dissolved in methanol and subjected to a treatment in an ultrasonic bath under stirring until potassium carbonate was completely dissolved (approximately 2.5 hours). Thereafter, solid matters were removed by filtration, the solvent was distilled off with a rotary evaporator, and a white solid matter thus formed was rinsed with dichloromethane and dried in vacuum, thereby synthesizing 1-methyl-5-mercapto-1,2,3,4-tetrazole potassium salt. The reaction yield at this step was 82%.

Subsequently, 1 mol of the compound thus synthesized was dissolved in methanol, to which 0.5 mol equivalent of iodine was added, followed by stirring the solution until iodine was dissolved. Thereafter, an excessive amount of water was added thereto to form a white precipitate, which was then collected by filtration, rinsed with water, and then dried in vacuum for 24 hours, thereby providing 5,5'-dithiobis(1-methyl-1H-tetrazole) as an oxidant of the redox pair. The reaction yield was 70%.

### Synthesis of 1-Methyl-5-mercapto-1,2,3,4-tetrazole Lithium Salt

1-Methyl-5-mercapto-1,2,3,4-tetrazole lithium salt was synthesized in the same manner as in the synthesis of 1-methyl-5-mercapto-1,2,3,4-tetrazole potassium salt except that lithium carbonate was used instead of potassium carbonate. The reaction yield was 48%.

### Synthesis of 1-Methyl-5-mercapto-1,2,3,4-tetrazole 1-Methyl-3-ethylimidazolium Salt

A methanol solution of 1 mol equivalent of 1-methyl-1,2,3,4-tetrazole-5-thiol dissolved therein and a methanol solution of 1 mol equivalent of 1-methyl-3-ethylimidazolium hydrogen carbonate (EMIm-HCO₃) were mixed and stirred for 3 hours, and then the solvent was distilled off with a rotary evaporator, thereby synthesizing 1-methyl-5-mercapto-1,2,3,4-tetrazole 1-methyl-3-ethylimidazolium salt in the form of liquid at ordinary temperature. The reaction yield was 98%.

### Production of Counter Electrode

An electrode of poly(3,4-ethylenedioxythiophene) doped with p-toluenesulfonic acid (PEDOT-PTS) was used as a counter electrode 9. Glass having an FTO film (produced by Asahi Glass Co., Ltd., 10 Ω per square or less) used as an electrode substrate 7 was cleaned under application of ultrasonic wave in an organic solvent, on which a reaction solution formed by dissolving 3,4-ethylenedioxythiophene, iron(III) tris-p-toluenesulfonate and dimethylsulfoxide at a weight ratio of 1/8/1 in n-butanol was applied by a spin coating method. The spin coating was performed at a rotation condition of 2,000 rpm for 30 seconds, and the concentration of 3,4-ethylenedioxythiophene in the solution was 0.1 M. Subsequently, the electrode substrate coated with the solution was heated for 5 minutes in a thermostat chamber held at 110°C for polymerization, followed by rinsing with methanol, thereby producing a counter electrode. The PEDOT thin film thus produced had a thickness of approximately 0.05 µm.

### Production of Solar Cell

An electrolytic solution injection hole having a diameter of 1 mm was provided by an electric drill at an appropriate position on the counter electrode 9 thus produced, which was then adhered to the electrode substrate 8 containing the transparent substrate 1 equipped with the transparent conductive film 2 and having the titanium oxide film 3 formed thereon (working electrode) with a thermoplastic sheet (Himilan 1652, a trade name, produced by Du Pont-Mitsui Polychemicals Co., Ltd., thickness: 25 µm) intervening between the electrodes under application of heat and pressure. Subsequently, one of the electrolytic solutions 1 to 3 prepared above was injected between the electrodes, and a glass plate having a thickness of 1 mm was placed on the electrolytic solution injection hole, on which an UV sealant (30Y-727, a product name, produced by Three Bond Co., Ltd.) was applied and sealed by irradiation of UV light at an intensity of 100 mW/cm² for 30 seconds, thereby producing a solar cell.

### Evaluation of Photoelectric Conversion Efficiency and Durability of Solar Cell

### (1) Evaluation of Solar Cell using Compound (A)

The solar cells thus produced were evaluated in the following manner. For the capability evaluation, the solar cell was evaluated for the load characteristics (I-V characteristics) with a potentiostat under irradiation with a solar simulator, XES-502S (available from Kansai Science Mechanical Co., Ltd.), having a xenon lamp equipped with an AM filter, under the irradiation condition of 100 mW/cm² after spectrum adjustment to AM 1.5 G. The evaluation of the capability of the solar cell was determined by the extent of the conversion efficiency η (%). The retention of the device capability in a dark place at room temperature was also evaluated.

The light irradiation intensity was calculated by comparing the integral value of irradiation light in a wavelength λ region of from 500 to 900 nm with that of the standard solar light by using a spectrum analyzer (LS-100, produced by EKO Instruments Co., Ltd.).

### Examples 1 to 4 and Comparative Example 1

The photoelectric conversion efficiencies η (%) of the solar cells immediately after production using the electrolytic solution 1 of a sulfide redox pair and the compounds (a) to (d) and the comparative dye are shown in Table 1. The relative values (%) of the conversion efficiency η with respect to the result of Comparative Example 1 as 100 and the retention of the device capability, i.e., the relative value (%) of the conversion efficiency η after 30 days with respect to the result immediately after production of the solar cell as 100, are also shown in Table 1.

**Table 1**

| | Dye | Electrolytic solution | Conversion efficiency η (%) | Relative value of device capability (%) | Retention of device capability 30 days after production (%) |
|---|---|---|---|---|---|
| Example 1 | compound (a) | 1 | 0.72 | 175 | 100 |
| Example 2 | compound (b) | 1 | 0.72 | 175 | 100 |
| Example 3 | compound (c) | 1 | 0.64 | 154 | 100 |
| Example 4 | compound (d) | 1 | 1.04 | 252 | 100 |
| Comparative Example 1 | comparative dye | 1 | 0.41 | 100 | 100 |

### Examples 5 and 6 and Comparative Example 2

The photoelectric conversion efficiencies η (%) of the solar cells using the electrolytic solution 2 of a sulfide redox pair and the compounds (a) and (c) and the comparative dye are shown in Table 2. The relative values (%) of the conversion efficiency η with respect to the result of Comparative Example 2 as 100 and the retention of the device capability, i.e., the relative value (%) of the conversion efficiency η after 30 days with respect to the result immediately after production of the solar cell as 100, are also shown in Table 2.

**Table 2**

| | Dye | Electrolytic solution | Conversion efficiency η (%) | Relative value of device capability (%) | Retention of device capability 30 days after production (%) |
|---|---|---|---|---|---|
| Example 5 | compound (a) | 2 | 0.32 | 118 | 100 |
| Example 6 | compound (c) | 2 | 0.33 | 119 | 100 |
| Comparative Example 2 | comparative dye | 2 | 0.27 | 100 | 100 |

It is understood from the results shown in Tables 1 and 2 that as compared to Comparative Examples 1 and 2 using the comparative dye, which was the dye shown in JP-A-2007-220412, Examples 1 to 6 using the sensitizing dyes of the invention were dominantly superior in the conversion efficiency and did not suffer capability deterioration even after 30 days from the production of the cells. It is expected that this is because the association of molecules of the dye is suppressed by the improvement of the molecular structure, and a suitable difference in potential between the redox potential of the sulfide redox pair and the HOMO level of the dye is provided.

### Example 7 and Comparative Example 3

The photoelectric conversion efficiencies η (%) of the solar cells immediately after production and after 45 days using the electrolytic solution 3 of an iodide redox pair and the compound (a) and the comparative dye are shown in Table 3. As the retention of the device capability, the relative value (%) of the conversion efficiency η after 45 days with respect to the result immediately after production of the solar cell as 100 is also shown in Table 3.

**Table 3**

| | Dye | Electrolytic solution | Conversion efficiency η (%) | | Retention of device capability 45 days after production (%) |
|---|---|---|---|---|---|
| | | | Initial | After 45 days | |
| Example 7 | compound (a) | 3 | 1.79 | 1.79 | 100 |
| Comparative Example 3 | comparative dye | 3 | 1.89 | 1.64 | 87 |

As shown in Table 3, Example 7 using the sensitizing dye of the invention was excellent in the retention of device capability as compared to Comparative Example 3 using the comparative dye, and was slightly inferior in the photoelectric conversion capability immediately after the production of the cell as compared to Comparative Example 3 but was superior thereto after 45 days. It is expected that this is because the association of molecules of the dye is suppressed by the improvement of the molecular structure.

As clear from the results, the photoelectric conversion devices using the sensitizing dyes of the invention are excellent in the photoelectric conversion capability for near infrared light and the stability of the device, as compared to the devices using ordinary dyes.

### (2) Evaluation of Solar Cell using Compound (B)

### Examples 8 to 10 and Comparative Example 4

The solar cells of Examples 8 to 10 and Comparative Example 4 immediately after production were evaluated in the same manner as above. Specifically, for the capability evaluation, the solar cell was evaluated for the load characteristics (I-V characteristics) with a potentiostat under irradiation with a solar simulator, XES-502S (available from Kansai Science Mechanical Co., Ltd.) having a xenon lamp equipped with an AM filter, under the irradiation condition of 100 mW/cm² after spectrum adjustment to AM 1.5 G. The evaluation of the capability of the solar cell was determined by the extent of the conversion efficiency η (%). The retention of the device capability in a dark place at room temperature was also evaluated. The light irradiation intensity was calculated by comparing the integral value of irradiation light in a wavelength λ region of from 500 to 900 nm with that of the standard solar light by using a spectrum analyzer (LS-100, produced by EKO Instruments Co., Ltd.). The photoelectric conversion efficiencies η (%) are shown in Table 4.

**Table 4**

| | Dye | Dye adsorption time (h) | Conversion efficiency η (%) | Retention of device capability 30 days after production (%) |
|---|---|---|---|---|
| Example 8 | compound (e) | 1 | 2.36 | 100 |
| Example 9 | compound (f) | 1 | 2.07 | 100 |
| Example 10 | compound (f) | 2 | 2.20 | 100 |
| Comparative Example 4 | comparative dye | 1 | 1.91 | 87 |

As shown in Table 4, Examples 8 to 10 using the sensitizing dyes of the invention were superior in the photoelectric conversion efficiency to Comparative Example 4 using the comparative dye, and did not suffer deterioration of the capability even after 30 days or more from the production of the cells. It is expected that this is because the association of molecules of the dye is suppressed by the improvement of the molecular structure as described above. The compound (f) used in Examples 9 and 10 had a long alkyl chain and thus exhibited a slow dye adsorption rate, but it was found that the device capability was enhanced by prolonging the dye adsorption time to 2 hours as in Example 10.

As described above, the sensitizing dye of the invention exhibits excellent photoelectric conversion characteristics in for near infrared light and excellent stability of the device capability, as compared to the ordinary dyes, and can be further enhanced in the photoelectric conversion characteristics by optimizing the dye adsorption conditions.

The photoelectric conversion device of the invention may be applied to a photoelectric conversion device, a light sensor and the like, which may be used indoors and outdoors.

## Claims

1. A compound having a structure represented by the following general formula (1) : 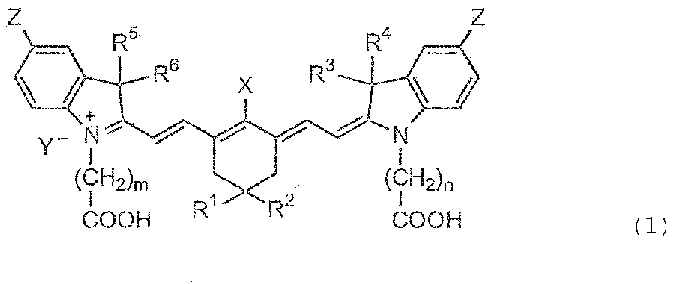 wherein R¹ and R², which may be the same as or different from each other, each represent a hydrogen atom, an alkyl group or an aryl group; R³ to R⁶, which may be the same as or different from each other, each represent an alkyl group having from 1 to 18 carbon atoms; X represents a halogen atom; Y represents a monovalent anion; Z represents an alkyl group, an alkoxy group or an aryl group when R³ to R⁶ each have from 1 to 5 carbon atoms, or a hydrogen atom, an alkyl group, an alkoxy group or an aryl group when R³ to R⁶ each have from 6 to 18 carbon atoms; and m and n, which may be the same as or different from each other, each represent an integer of from 1 to 12.

2. A sensitizing dye comprising a compound (A) represented by the general formula (1) wherein R³ to R⁶ each represent an alkyl group having from 1 to 12 carbon atoms; and Z represents an alkyl group, an alkoxy group or an aryl group.

3. A sensitizing dye comprising a compound (B) represented by the general formula (1) wherein R³ to R⁶ each represent an alkyl group having from 6 to 18 carbon atoms; and Z represents a hydrogen atom, an alkyl group, an alkoxy group or an aryl group.

4. A photoelectric conversion device comprising a semiconductor electrode, a counter electrode, and an electrolyte layer that is held between the electrodes, the semiconductor electrode containing the sensitizing dye according to claim 2 or 3 adsorbed thereon.

5. A dye sensitized solar cell comprising the photoelectric conversion device according to claim 4.
